# EUROPEAN PATENT APPLICATION

(11) **EP 1 938 842 A1**
(43) Date of publication of application: **02.07.2008**
(21) Application number: 06797249.7
(22) Date of filing: 01.09.2006
(51) Int. Cl.: A61K 47/04, A61K 9/20, A61K 47/32, A61K 47/36, A61K 47/38

(54) **METHOD FOR PREPARATION OF PHARMACEUTICAL COMPOSITION HAVING IMPROVED DISINTEGRADABILITY**

(30) Priority: 01.09.2005 JP 2005253305
(71) Applicant: Eisai R&D Management Co., Ltd., Tokyo 112-8088 (JP)
(72) Inventor: UEKI, Yosuke, Gifu, 5016195 (JP)
(74) Representative: HOFFMANN EITLE
(86) International application number: PCT/JP2006/317307
(87) International publication number: WO 2007/026864

(57) **Abstract**

There exists a strong desire both for pharmaceutical compositions which rapidly exhibit pharmacological effects without an increase in the size of the dosage form or a decline in quality due to interactions between a pharmaceutically active ingredient and the disintegrant, and also for a method of preparing such pharmaceutical compositions. Such a desire is especially acute with regard to, for example, preparations which contain a drug such as an analgesic or a quick-acting hypoglycemic drug that requires the rapid appearance of pharmacological effects following administration, preparations which have a high content of the pharmaceutically active ingredient, and preparations which contain two or more different active pharmaceutical ingredients. Thus, the object of the present invention is to improve the disintegratability of the pharmaceutical compositions without increasing the size of the dosage form and without a decline in quality due to interactions between the pharmaceutically active ingredient and the disintegrant. The present invention provides a method for preparing a pharmaceutical composition having a rapid disintegration time, comprising: blending, in the pharmaceutical composition containing a pharmaceutically active ingredient, at least one disintegrant and at least one water-soluble salt having a pH being from 3 to 9 in an aqueous solution of 2.5% concentration. The invention also provides a premix composition obtained by the preliminary mixture of a disintegrant with a water-soluble inorganic salt having a pH being from 3 to 9 in an aqueous solution of 2.5% concentration.

## Description

### Technical Field

The present invention relates to a method for improving disintegratability of drug products and producing pharmaceutical compositions having a rapid disintegration time by blending therein both a disintegrant and a water-soluble salt, and more specifically by blending therein both a disintegrant and a water-soluble inorganic salt having a pH being from 3 to 9 in an aqueous solution of 2.5% concentration. In particular, the invention relates to a method for improving the disintegratability of drug products by blending therein low-substituted hydroxypropyl cellulose and a water-soluble salt. Also, the invention relates to a premix composition obtained by the preliminary mixture of a disintegrant with a water-soluble inorganic salt having a pH being from 3 to 9 in an aqueous solution of 2.5% concentration.

### Background of Art

In order for a pharmaceutical composition to exhibit pharmacological effects, the pharmaceutically active ingredient included to the pharmaceutical composition must be absorbed into the body. Orally administered pharmaceutical compositions are generally absorbed in the body by passing through three stages: (1) disintegration of the pharmaceutical composition, (2) dissolution of the pharmaceutically active ingredient, and (3) absorption of the pharmaceutically active ingredient from the digestive tract. Hence, the pharmaceutical composition must disintegrate for the pharmacological effects to appear. For example, when a pharmaceutical composition such as a tablet does not rapidly disintegrate within the digestive tract following oral administration, the dissolution rate and the absorption rate of the pharmaceutically active ingredient decrease, leading to problems such as the following. A first problem is that a rapid pharmacological effect cannot be expected to appear following administration. This is an especially important concern in drugs that are required to manifest rapid pharmacological effects, such as analgesics (e.g., opioid drugs) and quick-acting hypoglycemic drugs (e.g., nateglinide). A second problem is the decline in pharmacological effects and the uncertainty of those effects (increased variability of pharmacological effects) owing to the decreased bioavailability of the drug. This latter concern is important in preparations which have a high drug content, preparations which contain a poorly soluble drug, and preparations which contain a poorly absorbed drug.

One general approach for improving the disintegratability of the pharmaceutical composition is a method that involves adding a disintegrant to the pharmaceutical composition. However, to achieve rapid disintegratability of the pharmaceutical composition, it is often necessary to add a large amount of disintegrant. In such cases, a number of problems arise: (1) compliance decreases as the size of the dosage form becomes larger, (2) productivity decreases as the size of the dosage form increases, and (3) the cost of the bulk materials for the drug product rises. Because increasing the drug content in the preparation is generally accompanied by an increase in the size of the dosage form and prolongation of the disintegration time, the foregoing problems are especially acute in preparations having a high drug content. In the case of drugs which, when formulated together with a disintegrant, undergo a decrease in chemical stability or experience a decline in release from the preparation, these problems appear due to limitations on the types of disintegrants that can be formulated in the pharmaceutical preparation. Examples of interactions between drugs and the disintegrant include oxidative decomposition of the drug due to the peroxide present in crospovidone, and electrostatic interactions between polyanionic disintegrants such as croscarmellose sodium and cations such as the acidic salt of basic drugs. Moreover, in preparations containing two or more different drugs, not only are there many instances where the particular disintegrants that may be used are limited by interactions between the drug and the preparation, because the drug content in the preparation is higher than in preparations containing only one drug, resolving the above problems often becomes even more difficult.

One way to overcome these problems is to develop novel disintegrants having a high disintegratability. However, the vast amounts of safety data that must be collected in order to employ such compounds in pharmaceutical and food products represents a very high barrier in terms of both time and cost. By contrast, if it were possible to find additives that, when employed together with disintegrants currently in common use, could enhance the disintegratability, improvements in disintegratability would be achievable without any accompanying drawbacks in terms of time and cost, which would be industrially beneficial. In particular, because improvements in the disintegratability would be achievable without a loss in the quality of the pharmaceutical product even in cases where drug-disintegrant interactions limit the disintegrants that can be used, such a development would have a very high industrial utility.

Examples of prior art relating to improvements in the disintegratability of pharmaceutical preparations using inorganic salts are given below. For example, Patent Document 1 discloses rapidly disintegrating tablets which contain a water-insoluble inorganic excipient and a disintegrant; Patent Document 2 discloses solid preparations containing a neutral or basic water-insoluble silicate, a water-insoluble phosphate, a water-insoluble metal oxide and a disintegrant; and Patent Document 3 discloses an improvement in the disintegratability of chitosan-containing tablets using sodium chloride alone. Patent Document 4 discloses a blowing agent containing citric acid and an alkaline earth metal carbonate, and relates to tablets having an improved disintegratability due to the generation of carbon dioxide within the digestive juices. Patent Document 5 discloses tablets of improved disintegratability which are obtained by incorporating a volatile excipient such as ammonium bicarbonate or ammonium carbonate, and sublimating the volatile salt by drying under applied heat and a vacuum so as to form porous tablets. In addition, Patent Document 6 discloses a method for improving the disintegratability of pressed compacts in which sodium chloride or potassium chloride has been added to a pharmaceutical composition; and Patent Document 7 discloses a composition containing a solid dispersion of a poorly soluble drug, in which composition the disintegratability has been improved with a substance such as sodium chloride which has an endothermic heat of dissolution. However, improvements in disintegratability achieved through the concomitant use of disintegrants and water-soluble inorganic salts such as sodium chloride have not been disclosed in these prior documents.
Patent Document 1: Japanese Patent Application (Published Japanese Translation of PCT International Publication) No. 2002-505269
Patent Document 2: Japanese Patent Application Laid-open No. H10-114655
Patent Document 3: Japanese Patent Application Laid-open No. H10-316576
Patent Document 4: Japanese Patent Application (Published Japanese Translation of PCT International Publication) No. 2002-509872
Patent Document 5: Japanese Patent Application (Published Japanese Translation of PCT International Publication) No. 2004-517859
Patent Document 6: Swiss Patent No. CH 656535
Patent Document 7: International Publication WO 98/29137

### Disclosure of Invention

### Problems to be Solved by the Invention

Accordingly, there exists a strong desire both for pharmaceutical compositions which rapidly exhibit pharmacological effects without an increase in the size of the dosage form due to a larger amount of disintegrant or a decline in quality due to interactions between the pharmaceutically active ingredient and the disintegrant, and also for a method of preparing such pharmaceutical compositions. Such a desire is especially acute with regard to preparations which contain a drug such as an analgesic or a quick-acting hypoglycemic drug that requires the rapid appearance of pharmacological effects following administration, preparations which have a high content of the pharmaceutically active ingredient, and preparations which contain two or more different pharmaceutically active ingredients.

It is therefore an object of the present invention is to provide a method for preparing pharmaceutical compositions of improved disintegratability without an increase in the size of the dosage form due to the addition of disintegrant and without a decline in quality due to interactions between the active pharmaceutical ingredient and the disintegrant. Another object of the invention is to provide premixed disintegrant compositions which are capable of improving disintegratability without an increase in the size of the dosage form due to the addition of disintegrant and without a decline in quality due to interactions between the pharmaceutically active ingredient and the disintegrant.

### Means for Solving the Problems

The inventors have conducted extensive studies in order to resolve the above problems. As a result, they have discovered that by employing a water-soluble salt, especially a water-soluble inorganic salt commonly used as a drug additive, such as sodium chloride or potassium chloride, together with a disintegrant such as low-substituted hydroxypropyl cellulose, the disintegratability of pharmaceutical preparations can be markedly improved. Moreover, the inventors have found that combinations of various water-soluble salts with various disintegrants improve pharmaceutical preparation disintegratability. These discoveries ultimately led to the present invention. With the inventive method of preparation, even when the disintegrants that may be used are limited owing to such reasons as interactions between the pharmaceutically active ingredient and the disintegrant, pharmaceutical compositions with excellent disintegratability can be prepared without an increase in the size of the dosage form or a decrease in quality on account of interactions between the pharmaceutically active ingredient and the disintegrant.

According to one aspect of the invention, there is provided a method for preparing pharmaceutical compositions, which method includes the step of blending, in a pharmaceutical composition containing a pharmaceutically active ingredient, at least one disintegrant and at least one water-soluble salt having a pH being from 3 to 9 in an aqueous solution of 2.5% concentration. By including both the disintegrant and the water-soluble salt, it is possible to achieve more rapid disintegration of the pharmaceutical composition than when either of these ingredients is included by itself.

The invention also provides a method for improving the disintegration time of a pharmaceutical composition by blending, in a pharmaceutical composition containing a pharmaceutically active ingredient, at least one disintegrant and at least one water-soluble salt having a pH being from 3 to 9 in an aqueous solution of 2.5% concentration.

According to a second aspect, the invention provides a premix composition lacking a pharmaceutically active ingredient, which composition includes at least one disintegrant and at least one water-soluble salt having a pH being from 3 to 9 in an aqueous solution of 2.5% concentration.

### Advantageous Effects of the Invention

The present invention improves the disintegratability of the pharmaceutical compositions without increasing the size of the dosage form and without a decline in quality due to interactions between the pharmaceutically active ingredient and the disintegrant, and thereby enables the preparation of the pharmaceutical compositions having a rapid disintegration time. Moreover, in the present invention, by using a premix composition lacking a pharmaceutically active ingredient, which composition includes at least one disintegrant and at least one water-soluble salt having a pH being from 3 to 9 in an aqueous solution of 2.5% concentration, the pharmaceutical composition having an improved disintegratability can be easily prepared by merely adding the premix composition to the formulation.

### Best Mode for Carrying Out the Invention

The embodiments presented below are to be considered in all respects as illustrative of the invention and not limitative. Various modifications and changes may be made thereto without departing from the spirit and scope of the invention.

The first aspect of the invention provides a method for preparing pharmaceutical compositions having a rapid disintegration time by blending, in the pharmaceutical composition containing a pharmaceutically active ingredient, at least one disintegrant and at least one water-soluble salt having a pH being from 3 to 9 in an aqueous solution of 2.5% concentration. First, the respective ingredients used in the inventive method of preparation are described.

### (Water-Soluble Salt):

In the present invention, the term "water-soluble salt" refers to a salt which is defined both by their pH in an aqueous solution of 2.5% concentration and by their solubility in purified water. The pH of an aqueous solution obtained by suspending or dissolving a water-soluble salt used in the present invention in water to a concentration of 2.5% is generally from 3 to 9, preferably from 4 to 8.5, and more preferably from 4.5 to 8. The salt is even more preferably a neutral salt (normal salt) (pH 5 to 8) of a strong acid and a strong base which has substantially no buffering ability. In the present invention, the water-soluble salt refers to a salt having a solubility in purified water of generally from 0.1 to 300 g/100 g of purified water, preferably from 0.5 to 200 g/100 g of purified water, more preferably from 1 to 100 g/100 g of purified water, and even more preferably from 2 to 50 g/100 g of purified water. In the water-soluble salt, the term "salt" refers to a salt formed by the complete or partial neutralization of "an organic acid or an inorganic acid" with "an organic base or an inorganic base". For example, bases which have not been neutralized (metal oxides, metal hydroxides), such as sodium hydroxide, aluminum hydroxide and magnesium oxide, are not encompassed by the salt used in the present invention. However, acidic salts such as sodium dihydrogen phosphate, which is a partially neutralized salt, and normal salts such as sodium chloride, which is a completely neutralized salt, are encompassed by the water-soluble salt used in the invention.

The water-soluble salt used in the invention is exemplified by water-soluble inorganic salts and water-soluble organic salts. In the present invention, the term "water-soluble inorganic salt" refers to a salt composed of a water-soluble inorganic acid and a water-soluble inorganic base. The term "water-soluble organic salt" refers to other salts; that is, to salts which include at least a water-soluble organic acid or a water-soluble organic base.

Examples of the water-soluble inorganic salts that may be used in the present invention include, but are not limited to, sodium chloride, sodium bromide, potassium chloride, potassium bromide, lithium chloride, disodium hydrogen phosphate, sodium dihydrogen phosphate, dipotassium hydrogen phosphate, potassium dihydrogen phosphate, ammonium chloride, ammonium bromide, aluminum chloride, calcium chloride, calcium bromide, magnesium chloride, magnesium bromide, barium chloride, barium bromide, sodium sulfate, magnesium sulfate, sodium bicarbonate, potassium bicarbonate, ammonium carbonate and the like. Examples of the water-soluble organic salts that may be used in the invention include, but are not limited to, organic salts such as sodium acetate, sodium oxalate, potassium acetate, sodium citrate, sodium dihydrogen citrate, disodium citrate, sodium succinate, monosodium succinate, sodium benzoate, disodium edetate, sodium erythorbate, sodium ascorbate, calcium acetate, potassium bitartrate, sodium tartrate, calcium lactate, sodium lactate, monosodium fumarate and the like; and amino acids such as glycine, aminoethanesulfonic acid, alanine, lysine hydrochloride, arginine hydrochloride, aspartic acid, glutamic acid and the like.

The water-soluble salt used in the invention encompasses water-soluble inorganic salts and water-soluble organic salts, although water-soluble inorganic salts are preferred. Of these, sodium chloride, magnesium chloride, calcium chloride, sodium bicarbonate, ammonium chloride and potassium chloride are more preferred; sodium chloride, magnesium chloride, sodium bicarbonate, potassium chloride and ammonium chloride are even more preferred; and sodium chloride is most preferred.

The amount of the water-soluble salt used in the invention that is added to the pharmaceutical composition is generally from 0.05 to 40 % by weight, preferably from 0.1 to 20 % by weight, more preferably from 0.2 to 10 % by weight, and even more preferably from 0.5 to 5 % by weight. At least one type of water-soluble salt used in the invention is added to the pharmaceutical composition, although two or more water-soluble salts may be added.

### (Disintegrant):

There are no particular limitations on the disintegrant used in the present invention, so long as it promotes the disintegration of the pharmaceutical composition owing to such properties as swelling in an aqueous solvent or the formation of water channels. However, disintegrants having a relatively weak swellability, such as cornstarch, are not included in the disintegrant used in the present invention. Specific examples of the disintegrant used in the present invention include sodium carboxymethyl starch, carboxymethylcellulose, carboxymethylcellulose calcium, low-substituted sodium carboxymethyl starch, low-substituted hydroxypropyl cellulose, hydroxypropyl starch, partly pregelatinized starch, croscarmellose sodium, crospovidone and the like. Of these, croscarmellose sodium, crospovidone, carboxymethylcellulose calcium, low-substituted hydroxypropyl cellulose and sodium carboxymethyl starch are preferred; croscarmellose sodium, carboxymethylcellulose calcium, low-substituted hydroxypropyl cellulose and sodium carboxymethyl starch are more preferred; and low-substituted hydroxypropyl cellulose is even more preferred.

Although there are no particular limitations on the amount of disintegrant included in the pharmaceutical composition according to the invention, the amount of the disintegrant is generally from 0.1 to 50 % by weight, preferably from 0.5 to 30 % by weight, more preferably from 1 to 20 % by weight, and even more preferably from 2 to 15 % by weight. At least one type of the disintegrant used in the invention may be included in the pharmaceutical composition, although it is possible to include two or more disintegrants.

In addition, there are no particular limitations on the combination of the disintegrant with the water-soluble salt in the pharmaceutical composition according to the invention, the combination of the disintegrant with the water-soluble salt is preferably a combination of low-substituted hydroxypropyl cellulose with sodium chloride, a combination of low-substituted hydroxypropyl cellulose with potassium chloride, or a combination of low-substituted hydroxypropyl cellulose with sodium bicarbonate. A combination of low-substituted hydroxypropyl cellulose with sodium chloride is more preferred.

Moreover, in the pharmaceutical composition according to the invention, there are no particular limitations on the amount of water-soluble salt included based on the disintegrant, the amount of the water-soluble salt is generally from 0.01 to10 parts by weight, preferably from 0.02 to 3 parts by weight, more preferably from 0.05 to 2 parts by weight, even more preferably from 0.10 to 1 part by weight, and most preferably from 0.15 to 0.5 part by weight, based on one part by weight of the disintegrant.

### (Pharmaceutically Active Ingredient):

There are no particular limitations on the pharmaceutically active ingredient used in the present invention, provided it exhibits a therapeutic effect when absorbed in the body. However, it is preferable for the pharmaceutically active ingredient to be electrically neutral or positively charged within the pharmaceutical preparation. An acidic active ingredient in a free form or an acidic salt of a basic active ingredient is more preferred. An acidic salt of a basic active ingredient is even more preferred. There are no particular limitations on the type of acidic salt of a basic active ingredient, provided it forms a pharmacologically acceptable salt with the pharmaceutically active ingredient. Examples thereof include hydrohalides (e.g., hydrofluorides, hydrochlorides, hydrobromides, hydroiodides), inorganic acid salts (e.g., sulfates, nitrates, perchlorates, phosphates, carbonates, bicarbonates), organic carboxylates (e.g., acetates, oxalates, maleates, tartrates, fumarates, citrates), organic sulfonates (e.g., mesylates, trifluoromethanesulfonates, ethanesulfonates, benzenesulfonates, tosylates, camphorsulfonates) and acidic amino acid salts (e.g., aspartates, glutaminates). Of these, hydrochlorides or tosylates of the pharmaceutically active ingredient are preferred, and tosylates of the active ingredient are more preferred. The acidic salts of basic active ingredients used in the invention also encompass cases in which the free form of a basic active ingredient and an acid (organic acid or inorganic acid) that have been separately included in the preparation elicit a neutralization reaction, and result in the formation of acid salt of the basic active ingredient within the pharmaceutical preparation.

Specific examples of the pharmaceutically active ingredient used in the present invention include antidementia agents such as donepezil hydrochloride, galantamine hydrobromide, rivastigmine tartrate, memantine hydrochloride and tacrine; drugs for treating diabetes such as nateglinide, metformin, α-glycosidase inhibitors (e.g., voglibose), sulfonylureas (e.g., glibenclamide, glimepiride), insulin resistance-enhancing agents (e.g., pioglitazone), dipeptidyl peptidase IV inhibitors (e.g., 3- but-2-ynyl-5-methyl-2-piperazin-1-yl-3,5-dihydro-4*H*-imidazo[4,5-d]pyridazin-4-one tosylate prepared by the method described in International Publication WO 03/104229); anti-anxiety drugs such as N-cyclopropylmethyl-7-(2,6-dimethoxy-4-methoxymethylphenyl)-2-ethyl-N-(tetrahydro-2*H*-pyran-4-ylmethyl)pyrazolo[1,5-a]pyridine-3-amine tosylate prepared by the method described in International Publication WO 02/088121; and vitamins such as ascorbic acid. Of these, the basic active ingredient is preferably a hydrochloride or tosylate of an antidementia agent or a drug for treating diabetes, and more preferably memantine chloride, donepezil hydrochloride, glibenclamide, 3-but-2-ynyl-5-methyl-2-piperazin-1-yl-3,5-dihydro-4*H*-imidazo[4,5-d]pyridazin-4-one tosylate, or N-cyclopropylmethyl-7-(2,6-dimethoxy-4-methoxoymethylphenyl)-2-ethyl-N-(tetrahydro-2*H*-pyran-4-ylmethyl)pyrazolo[1,5-a]pyridine-3-amine tosylate.

There are no particular limitations on the amount in which the pharmaceutically active ingredient having a pharmacological effect is included in the pharmaceutical composition according to the invention, the amount of the pharmaceutically active ingredient is generally from 10 to 99 % by weight, preferably from 20 to 97 % by weight, more preferably from 30 to 95 % by weight, and even more preferably from 40 to 95 % by weight, based on 100 % by weight of the overall pharmaceutical composition. Moreover, there are no particular limitations on the pharmaceutical composition according to the present invention, so long as it contains at least one type of the pharmaceutically active ingredient having a pharmacological effect which is intended to be rapidly released from the pharmaceutical composition.

### (Pharmaceutical Composition):

There are no particular limitations on the pharmaceutical composition according to the present invention, provided it is a pharmaceutical composition having an improved disintegratability and a rapid disintegration time owing to the addition of at least one disintegrant and at least one water-soluble salt having a pH being from 3 to 9 in an aqueous solution of 2.5% concentration. As used herein, the term "rapid disintegration time" means that the disintegration time is shortened relative to the addition of only a disintegrant or only a water-soluble salt to the pharmaceutical composition. The degree of shortening in the disintegration time is generally at least 10%, preferably at least 15%, more preferably at least 20%, and even more preferably at least 25%.

No particular limitation is imposed on the dosage form of the pharmaceutical composition according to the present invention. Preferred examples include dosage forms suitable for oral administration, such as tablets, capsules, granules and the like. Tablets are more preferred. The pharmaceutical composition according to the present invention is not subject to any particular limitation, so long as it includes at least one pharmaceutically active ingredient intended for rapid release from the pharmaceutical composition. Therefore, the pharmaceutical compositions which include only one type of pharmaceutically active ingredient having pharmacological effects and from which release of the active ingredient having pharmacological effects is slowed by a release-controlled coat, a release-controlled matrix and the like are not encompassed by the present invention.

There are no particular limitations on the distribution of the pharmaceutically active ingredient, the disintegrant, and the water-soluble salt having a pH being from 3 to 9 in an aqueous solution of 2.5% concentration within the pharmaceutical composition according to the invention, so long as these components are included in the pharmaceutical composition. However, it is preferable for each of the pharmaceutically active ingredient, the disintegrant and the water-soluble salt having a pH being from 3 to 9 in an aqueous solution of 2.5% concentration to be uniformly distributed within the pharmaceutical composition.

The pharmaceutical composition according to the present invention may comprises various pharmacologically acceptable carriers, such as excipients, lubricants, binders, disintegrants and the like, if necessary, may also comprises such additives as preservatives, colorants, sweeteners, plasticizers, film coating agents and the like. Examples of the excipients include sugars, sugar alcohols, starch, gelatinized starch, microcrystalline cellulose, light anhydrous silicic acid, synthetic aluminum silicate, magnesium aluminometasilicate and the like. Examples of the sugars include, but are not limited to, monosaccharides such as glucose, fructose and the like, and disaccharides such as maltose, lactose, sucrose, trehalose and the like. Examples of the sugar alcohols include, but are not limited to, mannitol, erythritol, inositol, sorbitol and the like. Examples of lubricants include, but are not limited to, magnesium stearate, calcium stearate, talc, sodium stearyl fumarate and the like. Examples of the binders include, but are not limited to, hydroxypropyl cellulose, methylcellulose, carboxymethyl cellulose sodium, hydroxypropyl methylcellulose, polyvinylpyrrolidone and the like. Examples of the disintegrants include, but are not limited to, carboxymethyl cellulose, carboxymethylcellulose calcium, croscarmellose sodium, sodium carboxymethyl starch, low-substituted hydroxypropyl cellulose and the like. Examples of the preservatives include, but are not limited to, p-oxybenzoic acid esters, chlorobutanol, benzyl alcohol, phenethyl alcohol, dehydroacetic acid, sorbic acid and the like. Examples of the colorants include, but are not limited to, water-insoluble lakes, natural colorants (e.g., β-carotene, chlorophyll and red iron oxide), yellow ferric oxide, red ferric oxide, black iron oxide and the like. Examples of the sweeteners include, but are not limited to, saccharin sodium, dipotassium glycyrrhizinate, aspartame, stevia and the like. Examples of the plasticizers include, but are not limited to, glycerol esters of fatty acids, triethyl citrate, propylene glycol, polyethylene glycol and the like. Examples of the film coating agents include, but are not limited to, hydroxypropyl methylcellulose, hydroxypropyl cellulose and the like. Film coatings are not limited to water-soluble film coatings. If necessary, a gastric coating or an enteric coating may be applied so as to give a pharmaceutical composition which is intended to rapidly release the pharmaceutically active ingredient following dissolution of the coating film. Alternatively, it is also acceptable not to apply a film coating.

### (Method of Preparation According to the Present Invention, Comprising Blending Water-Soluble Salt and Disintegrant Used in the present Invention):

Next, the method for preparation according to the present invention is described. The method of preparing a pharmaceutical composition comprises the step of blending, in the pharmaceutical composition containing a pharmaceutically active ingredient, at least one disintegrant and at least one water-soluble salt having a pH being from 3 to 9 in an aqueous solution of 2.5% concentration. The blending of the disintegrant or the water-soluble salt having a pH being from 3 to 9 in an aqueous solution of 2.5% concentration in the pharmaceutical composition according to the invention may be carried out in any one step or plurality of steps in the conventional preparation of the pharmaceutical composition. Moreover, the water-soluble salt and the disintegrant may be added in the same step or may each be added in separate steps.

There are no particular limitations on the method of preparation used in the present invention. For example, granules containing the pharmaceutically active ingredient may be formed by wet-granulating or dry-granulating powders containing the pharmaceutically active ingredient, the excipients etc. in a granulator. The granules thus obtained may, for example, be formed into tablets using a conventional tabletting machine. When wet granulation is carried out, the granulated granules obtained may be dried and, if necessary, rendered to a uniform size. In the method of preparation according to the present invention, the pharmaceutical composition in the form of tablets or the like may be produced by, for example, addition of the water-soluble salt before the granulation step, addition after the granulation step, or addition both before and after the granulation step. The disintegrant may be added in the same way as the water-soluble salt. The water-soluble salt may be added in the form of a powder, or as a solution or a suspension. When added as a powder, it is preferable to crush the salt into fine powders before addition.

In addition, the method for improving the disintegratability of the pharmaceutical compositions according to the present invention comprises blending, in the pharmaceutical composition containing a pharmaceutically active ingredient, at least one disintegrant and at least one water-soluble salt having a pH being from 3 to 9 in an aqueous solution of 2.5% concentration in the same manner as explained in the above-described method of preparation according to the present invention, thereby improving the disintegratability of the pharmaceutical composition, as compared to cases where only the disintegrant or the water-soluble salt alone is included in the pharmaceutical composition.

In another aspect of the present invention, there is provided a premix composition which lacks a pharmaceutically active ingredient, and which comprises at least one type of disintegrant and at least one type of water-soluble salt having a pH being from 3 to 9 in an aqueous solution of 2.5% concentration. By adding the premix composition according to the present invention to a composition containing a pharmaceutically active ingredient, the disintegratability of the pharmaceutical composition can easily be improved. That is, because the premix composition according to the present invention contains a water-soluble salt and a disintegrant that have been uniformly pre-mixed, the pharmaceutical compositions of improved disintegratability can be more conveniently obtained than when each of these ingredients is separately added, which is highly useful. Moreover, because the water-soluble salt having a pH being from 3 to 9 in an aqueous solution of 2.5% concentration and the disintegrant are uniformly distributed, even in cases where a powder is added as a preparation additive, there is no need for the powder to be finely milled. The term "uniformity" refers herein not to uniformity at the molecular level. Rather, it may refer to, for example, a physical mixture of the finely milled water-soluble salt and disintegrant (dry mixture), a form obtained by layering the water-soluble salt onto the surface of the disintegrant, or a form obtained by spray drying a suspension or solution of the disintegrant and the water-soluble salt.

There are no particular limitations on the combination of the disintegrant and the water-soluble salt having a pH being from 3 to 9 in an aqueous solution of 2.5% concentration according to the present invention. Preferable examples of the disintegrant include croscarmellose sodium, crospovidone, carboxymethylcellulose calcium, low-substituted hydroxypropyl cellulose or sodium carboxymethyl starch; more preferably croscarmellose sodium, carboxymethylcellulose calcium, low-substituted hydroxypropyl cellulose or sodium carboxymethyl starch; and even more preferably low-substituted hydroxypropyl cellulose. Preferable examples of the water-soluble salt include a water-soluble inorganic salt; more preferably sodium chloride, magnesium chloride, calcium chloride, sodium bicarbonate, ammonium chloride or potassium chloride; even more preferably sodium chloride, magnesium chloride, sodium bicarbonate, potassium chloride or ammonium chloride; and most preferably sodium chloride. Preferred combinations of the disintegrant with the water-soluble salt having a pH being from 3 to 9 in an aqueous solution of 2.5% concentration include a combination of low-substituted hydroxypropyl cellulose with sodium chloride, a combination of low-substituted hydroxypropyl cellulose with potassium chloride, and a combination of low-substituted hydroxypropyl cellulose with sodium bicarbonate. A combination of low-substituted hydroxypropyl cellulose with sodium chloride is most preferred.

In the present invention, although there are no particular limitations on the average particle size of the premix composition which comprises at least one water-soluble salt having a pH being from 3 to 9 in an aqueous solution of 2.5% concentration and at least one disintegrant but lacks a pharmaceutically active ingredient, the average particle size is generally from 1 to 1,000 µm, preferably from 5 to 500 µm, and more preferably from 10 to 250 µm. In the present invention, the ratio of the water-soluble salt to the disintegrant in the premix composition which comprises at least one water-soluble salt having a pH being from 3 to 9 in an aqueous solution of 2.5% concentration and at least one disintegrant but lacks a pharmaceutically active ingredient is generally from 0.01 to 10 parts by weight, preferably from 0.02 to 3 parts by weight, more preferably from 0.05 to 2 parts by weight, even more preferably from 0.10 to 1 part by weight, and most preferably from 0.15 to 0.5 part by weight, based on one part by weight of the disintegrant. Moreover, in the present invention, the premix composition which comprises at least one water-soluble salt having a pH being from 3 to 9 in an aqueous solution of 2.5% concentration and at least one disintegrant but lacks the pharmaceutically active ingredient may be used by blending them alone into the pharmaceutical composition, although disintegrants, water-soluble salts, excipients and other ingredients may also be added where necessary.

The pharmaceutical composition according to the present invention may be produced by, for example, the following method.

A suitable amount of purified water is added to 77.8 g of 3-but-2-ynyl-5-methyl-2-piperazin-1-yl-3,5-dihydro-4*H*-imidazo[4,5-d]pyridazin-4-one tosylate, which is a dipeptidylpeptitase IV inhibitor, prepared by the method described in International Publication WO 03/104229, 8.92 g of mannitol, 14.10 g of cornstarch, 21.15 g of low-substituted hydroxypropyl cellulose (Shin-Etsu Chemical) and 3.53 g of hydroxypropyl cellulose (Nippon Soda), and granulation is carried out in a stirring granulator. The granulated granules are subsequently dried by heating in a thermostatic chamber, then rendered to a uniform size. Next, 10 g of microcrystalline cellulose, 2 g of sodium chloride and 1 g of magnesium stearate are added per 89 g of the sized granules and mixed, following which the mixture is formed into tablets using a single-punch tabletting machine, enabling tablets having a weight of 239.7 mg and a diameter of 8.5 mm to be obtained. In addition, a water-soluble film composed primarily of hydroxypropyl methyl-cellulose or the like may be applied to the tablets using a coating machine.

Alternatively, the pharmaceutical composition according to the present invention may be prepared by, for example, the following method.

A solution obtained by dissolving 3.53 g of hydroxypropyl cellulose (Nippon Soda) and 2 g of sodium chloride in a suitable amount of purified water is added to 77.8 g of 3-but-2-ynyl-5-methyl-2-piperazin-1-yl-3,5-dihydro-4*H*-imidazo[4,5-d]pyridazin-4-one tosylate, 8.92 g of mannitol, 14.10 g of cornstarch and 21.15 g of low-substituted hydroxypropyl cellulose (Shin-Etsu Chemical), and granulation is carried out in a stirring granulator. The granulated granules are subsequently dried by heating in a thermostatic chamber, then rendered to a uniform size. Next, 10 g of microcrystalline cellulose and 1 g of magnesium stearate are added per 91 g of the sized granules and mixed, following which the mixture is formed into tablets using a single-punch tabletting machine, enabling tablets having a weight of 239.7 mg and a diameter of 8.5 mm to be obtained. In addition, a water-soluble film composed primarily of hydroxypropyl methyl-cellulose or the like may be applied to the tablets using a coating machine.

### EXAMPLES

Examples are given below to more fully illustrate the present invention, but are not intended to limit the scope of the invention.

### 1. Synergistic Effects of Low-Substituted Hydroxypropyl Cellulose (hereinbelow sometimes referred to as "L-HPC") with Sodium Chloride (hereinbelow sometimes referred to as "NaCl")

### Test Example 1:

Using the dipeptidyl peptidase IV inhibitor-containing tablets prepared in Examples 1 to 2 and Comparative Examples 1 to 6, the disintegration times were measured in accordance with the disintegration test method described in the Pharmacopoeia of Japan (test fluid: water; auxiliary disk not used). The results are shown in Tables 1 and 2.

The disintegration time in Comparative Example 1, where neither sodium chloride nor L-HPC was included, was 23.9 minutes; the disintegration times in Comparative Examples 2 to 4, where 5%, 10% or 20% of sodium chloride was added, were from 20.6 to 21.7 minutes; and the disintegration times in Comparative Examples 5 and 6, where 10% or 20% of L-HPC was added to Comparative Example 1, were from 17.8 to 18.3 minutes. Hence, even when sodium chloride or L-HPC was added in a high concentration of 20%, a sufficient disintegratability improving effect was not obtained. By contrast, the disintegration times in Examples 1 and 2, where sodium chloride and L-HPC were added in a combined amount of 10% at a proportion of 1:3 or 2:2, were respectively 8.2 minutes and 11.6 minutes. These results showed a distinct improvement in the disintegration time, as compared to the results obtained in Comparative Examples 1 to 6. Such improvement effects were even more striking than when 20% of the respective ingredients were added. Hence, the inclusion of both L-HPC and sodium chloride clearly had a synergistic disintegratability improving effect. In the tables, "St-Mg" refers to magnesium stearate.

### Table 1

**Table 1**

| | Active Ingredient-Containing Granules 1 | Active Ingredient-Containing Granules 2 | Active Ingredient-Containing Granules 3 | Active Ingredient-Containing Granules 4 | |
|---|---|---|---|---|---|
| Dipeptidyl peptidase IV inhibitor* | 10.00g | 77.80g | 2.593g | 2.593g | |
| Mannitol | 5.00g | 8.92g | 0.509g | 0.415g | |
| Cornstarch | - | 14.10g | 0.470g | 0.470g | |
| L-HPC | - | 21.15g | 0.705g | 0.705g | |
| NaCl | - | - | - | 0.094g | |
| HPC-L | 0.50g | 3.53g | 0.141g | 0.141g | |
| | | | | | |

| | Active Ingredient-Containing Granules 5 | Active Ingredient-Containing Granules 6 | Active Ingredient-Containing Granules 7 | Active Ingredient-Containing Granules 8 | Active Ingredient-Containing Granules 9 |
|---|---|---|---|---|---|
| Anti-anxiety drug** | 2.00g | - | - | - | - |
| Ascorbic acid | - | 3.00g | - | - | - |
| Glibenclamide | - | - | 2.00g | - | - |
| Donepezii hydrochloride | - | - | - | 1.00g | - |
| Memantine hydrochloride | - | - | - | - | 1.00g |
| Mannitol | 2.00g | 1.00g | 2.00g | 1.00g | 1.00g |
| HPC-L | 0.12g | 0.12g | 0.12g | 0.06g | 0.06g |

| | | | | | |
|---|---|---|---|---|---|
| * 3-But-2-ynyl-5-methyl-2-piperazin-1-yl-3,5-dihydro-4*H*-imidazo[4,5-d]pyridazin-4-one tosylate ** N-Cyclopropylmethyl-7-(2,6-dimethoxy-4-methoxymethylphenyl)-2-ethyl-N-(tetrahydro-2*H*-pyran-4-ylmethyl) pyrazolo[1,5-a]pyridine-3-amine tosylate | | | | | |

### Table 2

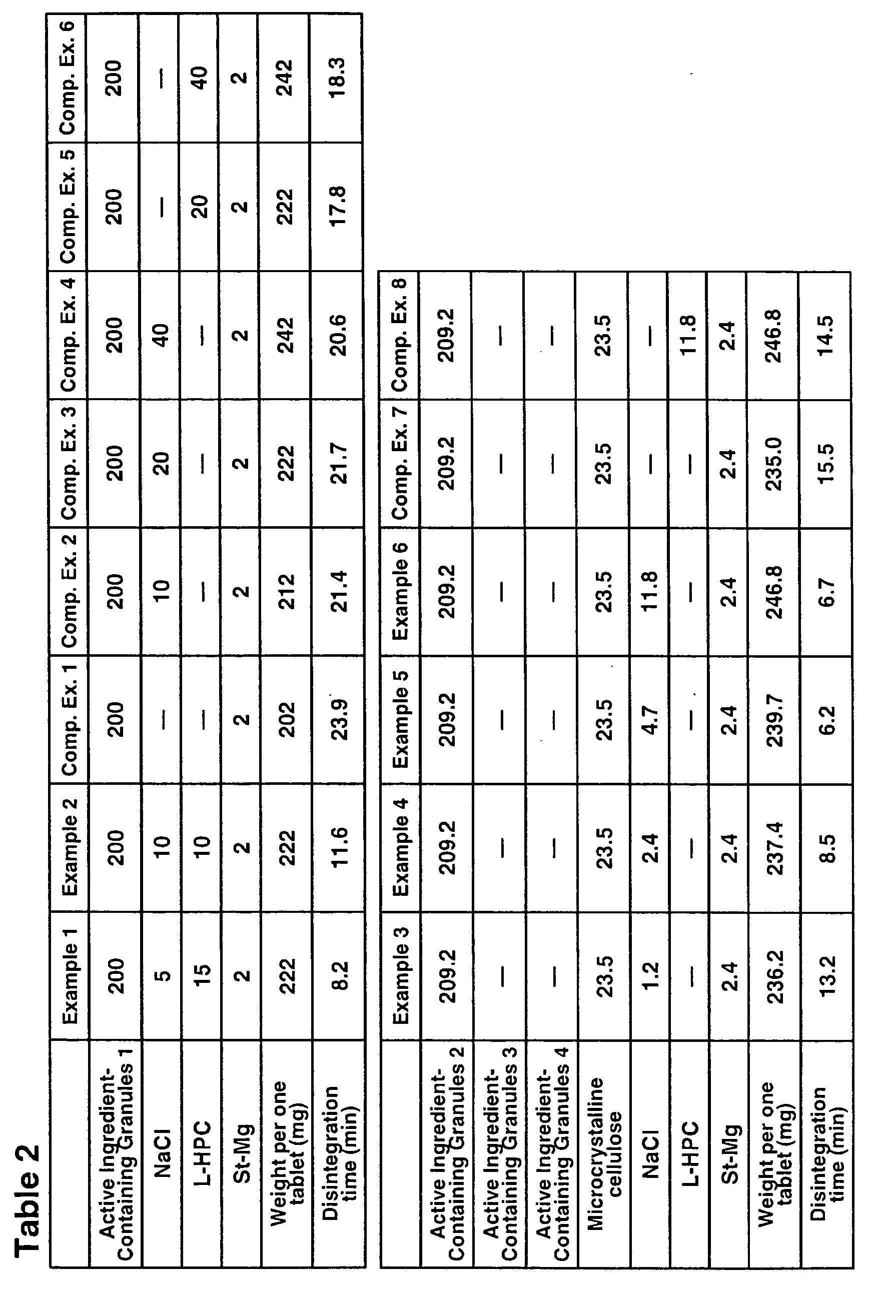

### Test Example 2:

A test was conducted on the effects of the sodium chloride content on the disintegration time.

Using the dipeptidyl peptidase IV inhibitor-containing tablets prepared in Examples 3 to 6 and Comparative Examples 7 and 8, the disintegration times were measured in accordance with the disintegration test method described in the Pharmacopoeia of Japan (test fluid: water; auxiliary disk not used) (see Table 2). As a result, as compared to a disintegration time in Comparative Example 7 which included L-HPC being 15.5 minutes, the disintegration times in Examples 3 to 6 which included 0.5%, 1%, 2% or 5% of sodium chloride were respectively 13.2 minutes, 8.5 minutes, 6.2 minutes and 6.7 minutes. Hence, the disintegration time improved as the amount of sodium chloride added was increased. In Comparative Example 8, where 5% of L-HPC was added to Comparative Example 7, the disintegration time was 14.5 minutes; hence, disintegration time improving effects were not observed in these examples. These results demonstrated that the synergistic disintegratability-enhancing effects of sodium chloride and L-HPC can be achieved with the addition of a small amount of sodium chloride.

### Test Example 3:

A test was conducted on the effects of the manner of sodium chloride blending on the disintegration time.

Using the dipeptidyl peptidase IV inhibitor-containing tablets prepared in Example 7 and Comparative Example 9, the disintegration times were measured in accordance with the disintegration test method described in the Pharmacopoeia of Japan (test fluid: water; auxiliary disk not used) (see Table 3). As a result, the disintegration times for Comparative Example 9 in which the granulated granules did not contain sodium chloride (Active Ingredient-Containing Granules 3 were used) and for Example 7 in which the granulated granules contained 2% of sodium chloride (Active Ingredient-Containing Granules 4 were used) were respectively 21.2 minutes and 9.2 minutes, demonstrating a disintegratability improving effect from the addition of sodium chloride. Regardless of the method of blending sodium chloride, i.e., regardless of whether sodium chloride is included into the granulated granules (intra-granularly) or as an external additive outside of the granulated granules (extra-granularly) (Test Examples 1 and 2), the results demonstrated that the synergistic disintegratability improving effects can be achieved with sodium chloride and L-HPC.

### Table 3

**Table 3**

| | Example 7 | Comp. Ex. 9 |
|---|---|---|
| Active Ingredient-Containing Granules 3 | - | 220.9 |
| Active ingredient-Containing Granules 4 | 220.9 | - |
| Microcrystalline cellulose | 11.8 | 11.8 |
| NaCl | - | - |
| L-HPC | - | - |
| St-Mg | 2.4 | 2.4 |
| Weight per one tablet (mg) | 235.0 | 235.0 |
| Disintegration time (min) | 9.2 | 21.2 |

### 2. Synergistic Effects of Various Disintegrants with Sodium Chloride

### Test Example 4:

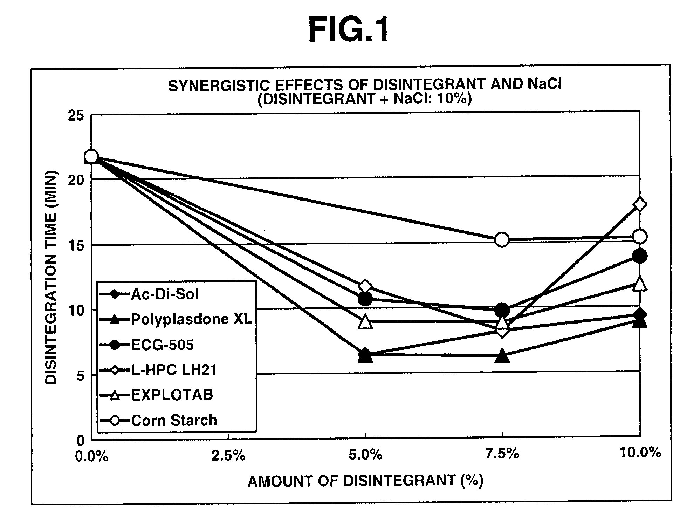

Using the dipeptidyl peptidase IV inhibitor-containing tablets prepared in Examples 8 to 15 and Comparative Examples 3 and 10 to 15, the disintegration times were measured in accordance with the disintegration test method described in the Pharmacopoeia of Japan (test fluid: water; auxiliary disk not used). The results are shown in Tables 4 and 5, and FIG. 1. It was found that the disintegration time improved markedly in Examples 8 to 15, which were pharmaceutical compositions containing croscarmellose sodium (sometimes indicated below as "Ac-di-sol"), crospovidone (sometimes indicated below as "polyplasdone XL"), carboxymethylcellulose calcium (sometimes indicated below as "ECG-505") or sodium carboxymethyl starch (sometimes indicated below as "EXPLOTAB") and containing also sodium chloride, as compared to cases where sodium chloride or one of the respective disintegrants was added alone. However, in Comparative Examples 14 and 15, where cornstarch, which has a weak swellability, was used as the disintegrant, the disintegration time remained about the same; that is, a synergistic effect owing to use together with sodium chloride was not confirmed. The synergistic effects on the disintegration time by croscarmellose sodium, crospovidone, carboxymethylcellulose calcium and sodium carboxymethyl starch when used together with sodium chloride were thus apparent.

### Table 4

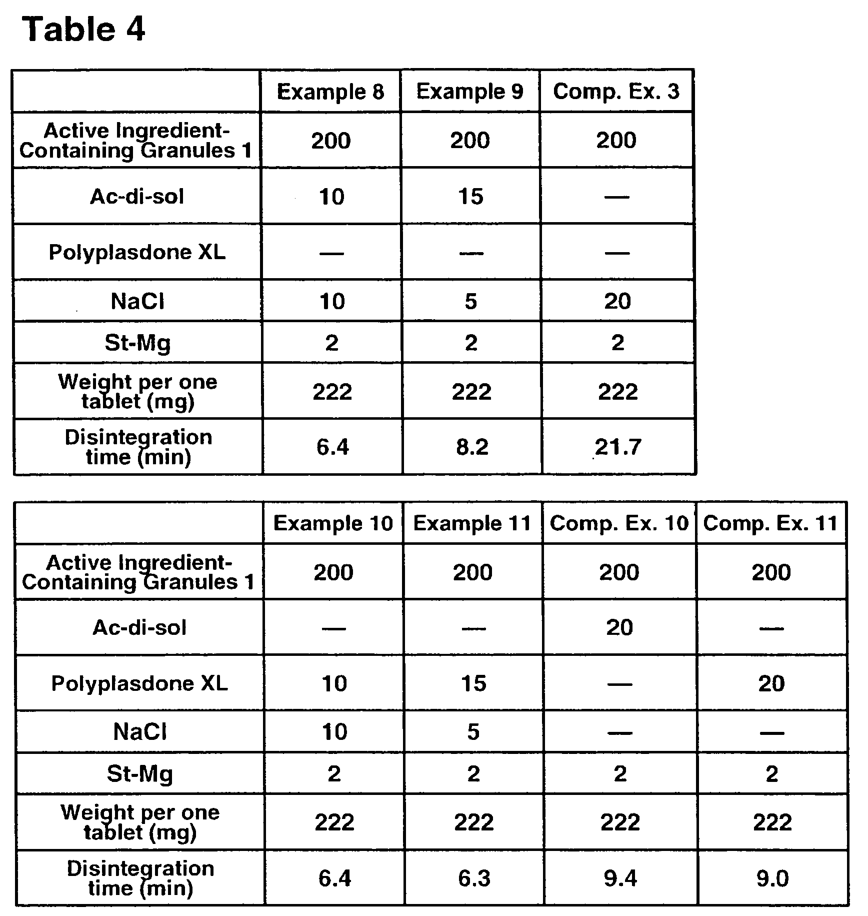

### Table 5

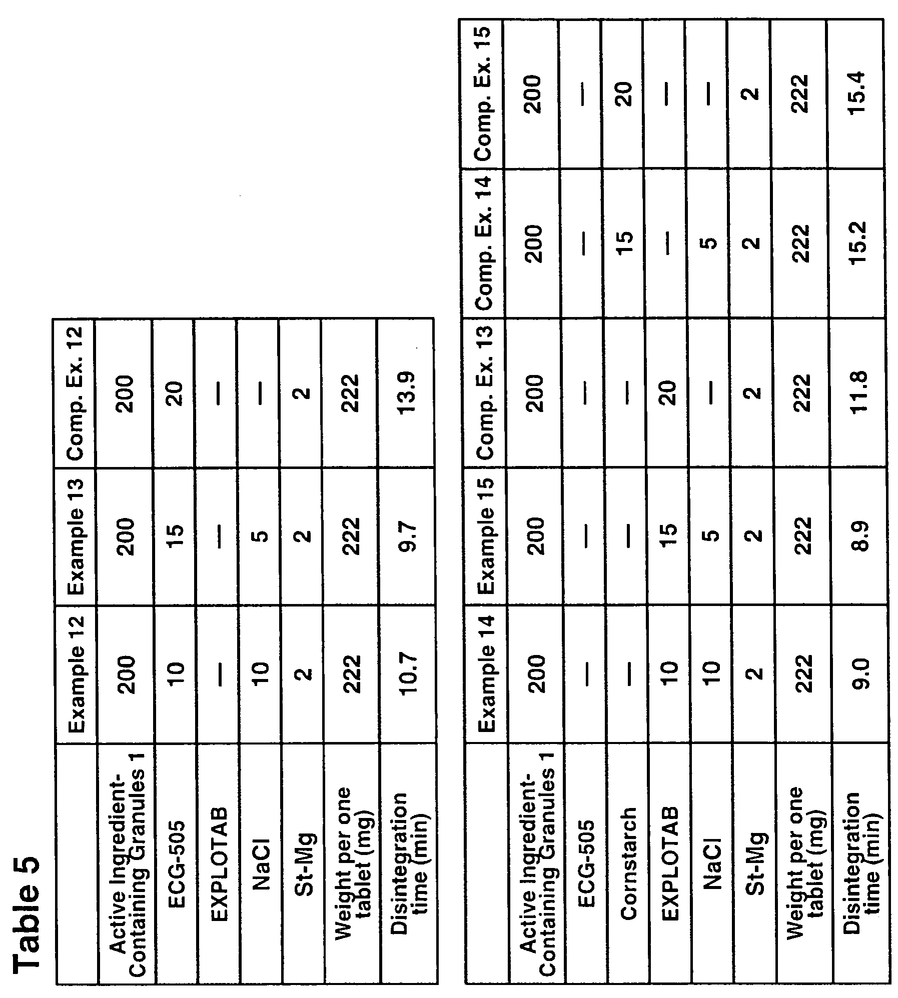

### 3. Synergistic Effects of Low-Substituted Hydroxypropyl Cellulose (L-HPC) with Various Water-Soluble Salts

### Test Example 5:

Using the dipeptidyl peptidase IV inhibitor-containing tablets prepared in Examples 16 to 25 and Comparative Examples 5 and 16 to 26, the disintegration times were measured in accordance with the disintegration test method described in the Pharmacopoeia of Japan (test fluid: water; auxiliary disk not used) (see Table 6). As a result, it was confirmed that, in each of Examples 16 to 25, which were pharmaceutical compositions containing a water-soluble salt other than anhydrous sodium carbonate, i.e., a water-soluble salt having a pH being from 3 to 9 in an aqueous solution of 2.5% concentration, the disintegration time improved markedly, as compared to cases in which L-HPC or one of the respective water-soluble salts was added alone. Moreover, this disintegratability improving effect was also confirmed for anhydrous calcium chloride, which has a heat of dissolution that is exothermic (Examples 18 and 19), demonstrating that the disintegratability improving effect does not depend on the absorption or generation of heat during dissolution of the water-soluble salt.

### Table 6

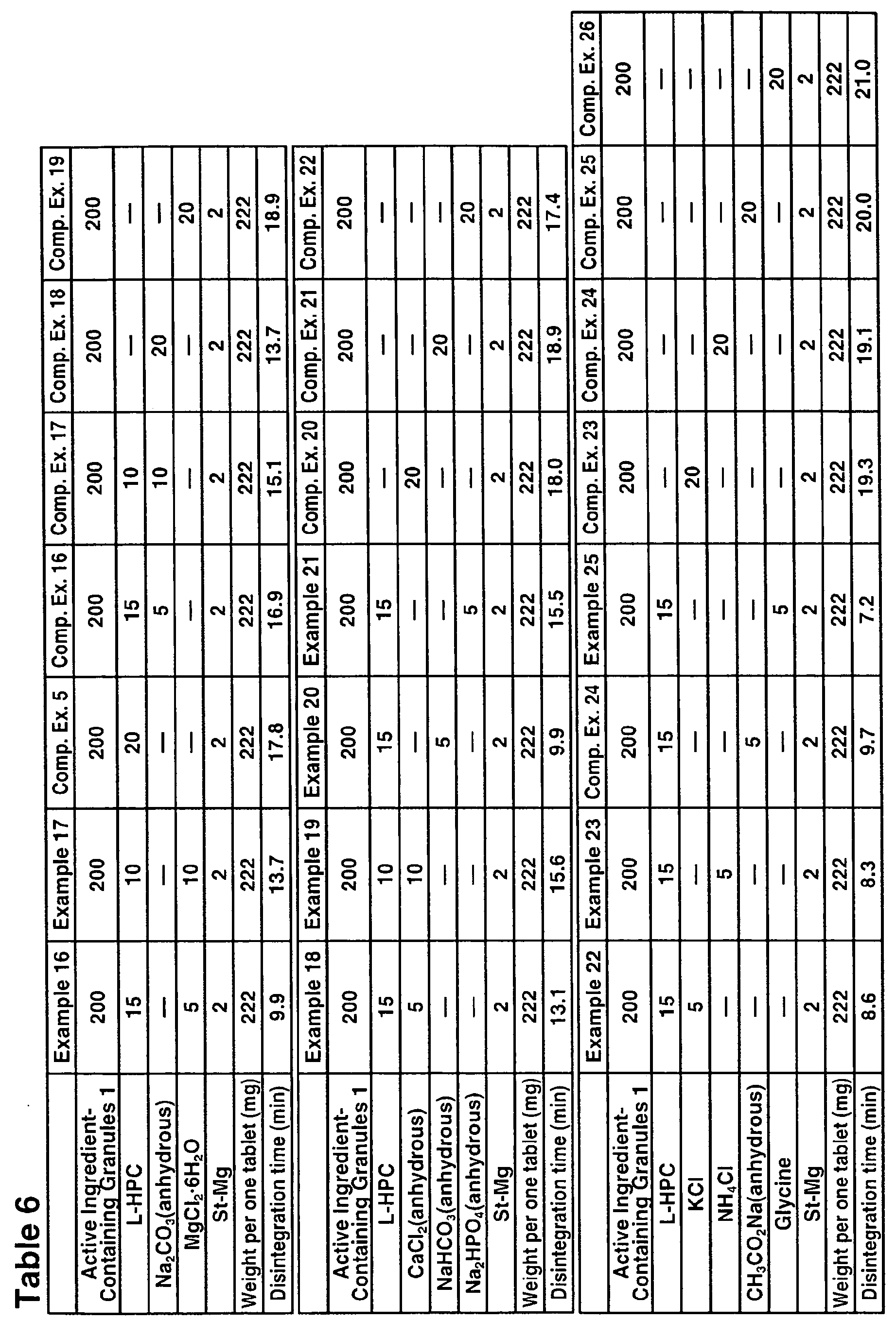

### 4. pH Effects of Water-Soluble Salts

### Test Example 6:

The influence on the disintegratability improving effect by the pH of various water-soluble salts which exhibit synergistic effects together with L-HPC was evaluated.

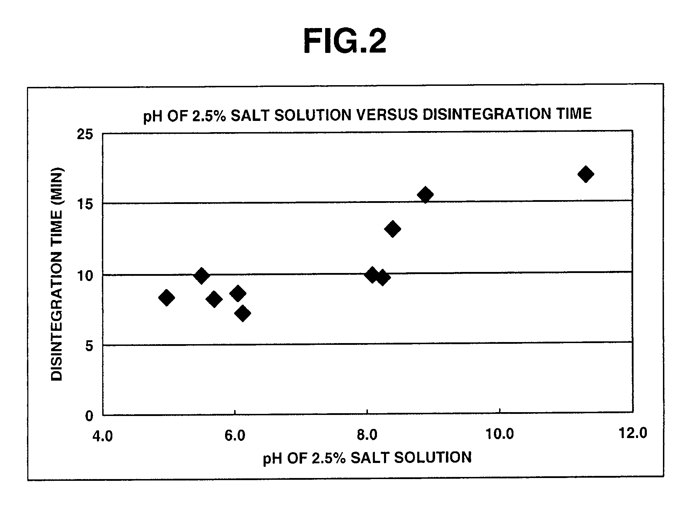

The pH values of 2.5 wt % aqueous solutions of the ten types of salts used in Test Examples 1 and 5 dissolved in purified water were measured. Those results are shown in Table 7. FIG. 2 shows the relationship between the pH values of the above 2.5% aqueous solutions and the disintegration time of tablets obtained by adding L-HPC/salt in a ratio of 7.5%/2.5% to the pharmaceutically active ingredient-containing granules (Comparative Example 16, and Examples 1, 16, 18 and 20 to 25).

It became apparent from the above results that the disintegratability improving effect weakens as the pH of the 2.5 wt % aqueous solution of the respective water-soluble salts rises. These results showed that the aqueous solutions prepared by suspending or dissolving the water-soluble salts used in the present invention to a concentration of 2.5% in water have a pH being generally from 3 to 9, preferably from 4 to 8.5, more preferably from 4.5 to 8. It became apparent that the water-soluble salt used in the present invention is most preferably a neutral salt (normal salt) of a strong acid and a strong base which has substantially no buffering ability (pH 5 to 8).

### Table 7

**Table 7**

| | Salt added | pH of 2.5% salt solution | Disintegration time (min) |
|---|---|---|---|
| Comparative Example 16 | Na₂CO₃(anhydrous) | 11.31 | 16.9 |
| Example 1 | NaCl | 5.69 | 8.2 |
| Example 16 | MgCl₂·6H₂O | 5.50 | 9.9 |
| Example 18 | CaCl₂(anhydrous) | 8.40 | 13.1 |
| Example 20 | NaHCO₃(anhydrous) | 8.09 | 9.9 |
| Example 21 | Na₂HPO₄(anhydrous) | 8.90 | 15.5 |
| Example 22 | KCI | 6.05 | 8.6 |
| Example 23 | NH₄Cl | 4.97 | 8.3 |
| Example 24 | CH₃CO₂Na(anhydrous) | 8.25 | 9.7 |
| Example 25 | Glycine | 6.12 | 7.2 |

### Test Example 7:

The disintegratability improving effects in combinations of various disintegrators with various water-soluble salts were evaluated.

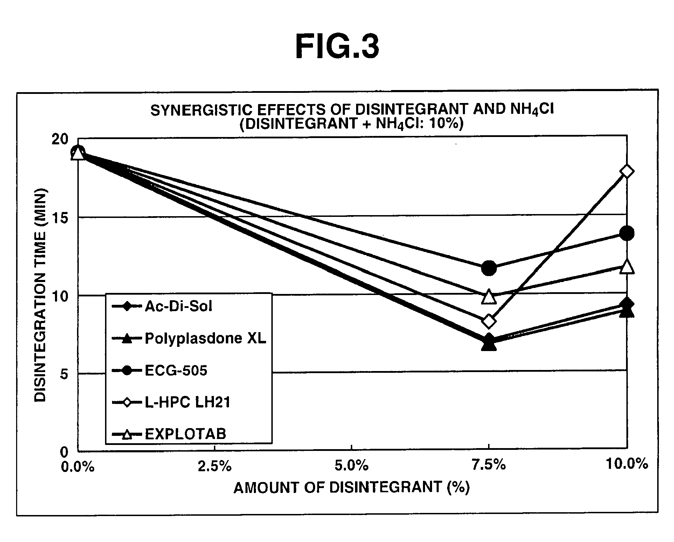

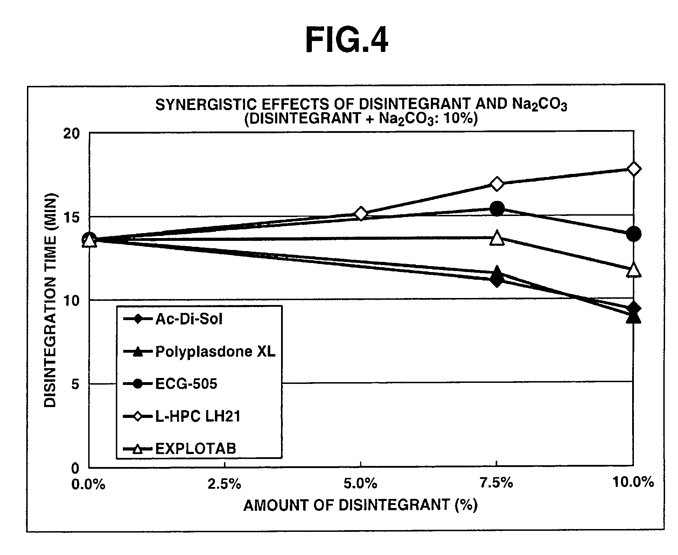

Using the dipeptidyl peptidase IV inhibitor-containing tablets prepared in Examples 26 to 29 and Comparative Examples 10 to 13, 18, 24 and 27 to 30, the disintegration times were measured in accordance with the disintegration test method described in the Pharmacopoeia of Japan (test fluid: water; auxiliary disk not used). The results are shown in Table 8 and FIGS. 3 and 4. As a result, when ammonium chloride, which is a salt that exhibits acidity in an aqueous solution, was used as the water-soluble salt, synergistic disintegratability improving effects owing to concomitant use with a disintegrator were confirmed in the respective tablets obtained in Examples 26 to 29. However, when anhydrous sodium carbonate, which is a salt that exhibits basicity in an aqueous solution, was used as the water-soluble salt, synergistic disintegratability improving effects owing to concomitant use with a disintegrator were not confirmed in the respective tablets obtained in Comparative Examples 27 to 30. From Test Examples 4 to 6 and the foregoing results, it was apparent that, even for disintegrators other than L-HPC, concomitant use together with a water-soluble salt that forms an aqueous solution which exhibits a pH in a range from weakly acidic to close to neutral results in a synergistic improvement in the disintegratability.

### Table 8

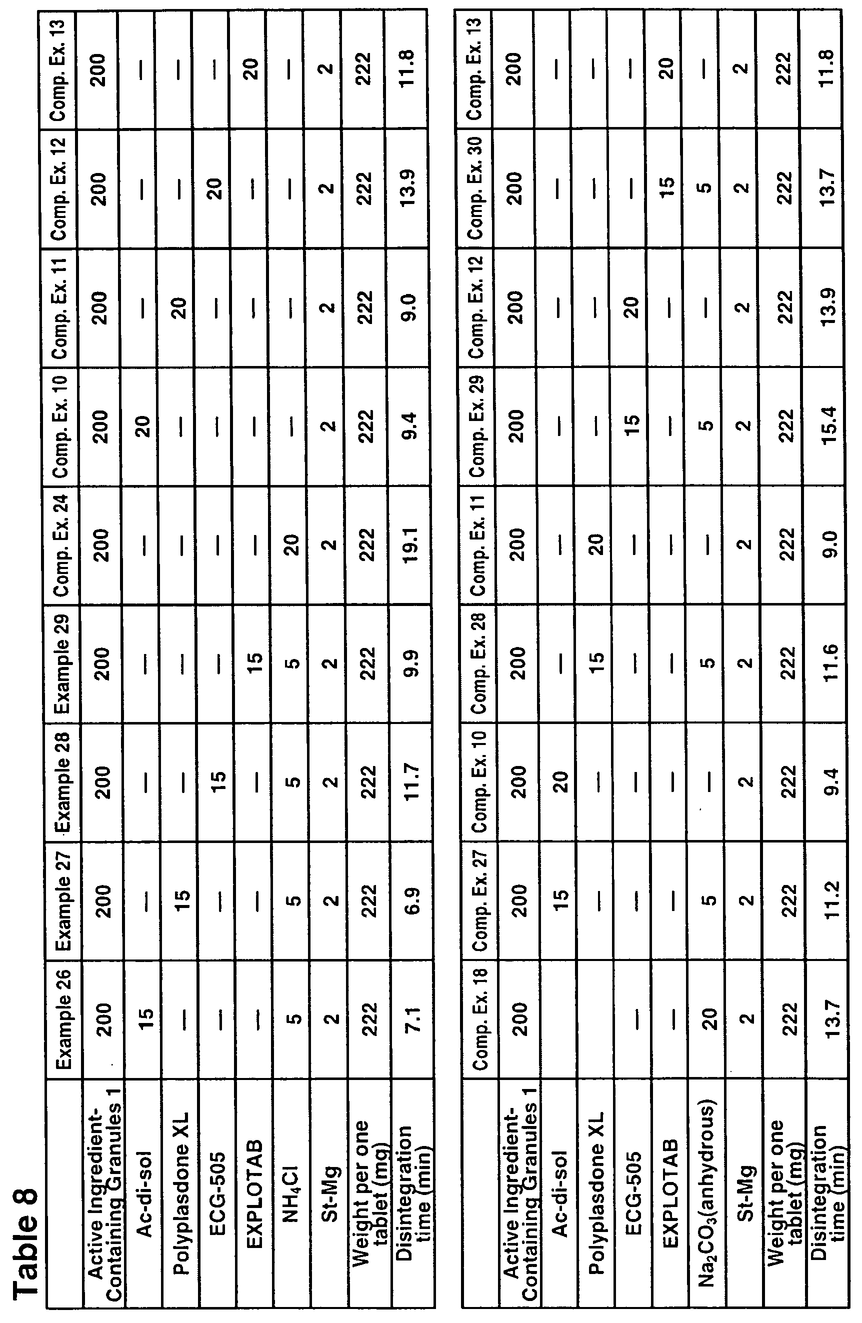

### 5. Disintegratability Improving Effects of Blending a Disintegrator and a Water-Soluble Inorganic Salt on Various Pharmaceutically Active Ingredients

### Test Example 8:

The disintegratability improving effects of a disintegrator and a water-soluble inorganic salt on various pharmaceutically active ingredients were evaluated.

Using the various pharmaceutically active ingredient-containing tablets prepared in Examples 30 to 34 and Comparative Examples 31 to 42, the disintegration times were measured in accordance with the disintegration test method described in the Pharmacopoeia of Japan (test fluid: water; auxiliary disk not used). The results are shown in Table 9. As a result, synergistic disintegratability improving effects due to the concomitant use of sodium chloride and L-HPC were observed in each of the tablets of Examples 30 to 34 which contained various pharmaceutically active ingredients (N-cyclopropylmethyl-7-(2,6-dimethoxy-4-methoxymethylphenyl)-2-ethyl-N-(tetrahydro-2*H*-pyran-4-ylmethyl)pyrazolo[1,5-a]pyridine-3-amine tosylate prepared by the method described in International Publication WO 02/088121, ascorbic acid, glibenclamide, donepezil hydrochloride or memantine hydrochloride). However, when anhydrous sodium carbonate and L-HPC were used together in a glibenclamide-containing tablet, as shown in Comparative Example 37, the disintegration time was slower than in Comparative Example 35 in which L-HPC alone was added, and so a disintegratability improving effect was not observed. From the above, it was apparent that, in various pharmaceutically active ingredients, the disintegratability is improved by adding a disintegrator and at least one type of water-soluble salt having a pH being from 3 to 9 in an aqueous solution at a concentration of 2.5%.

### Table 9

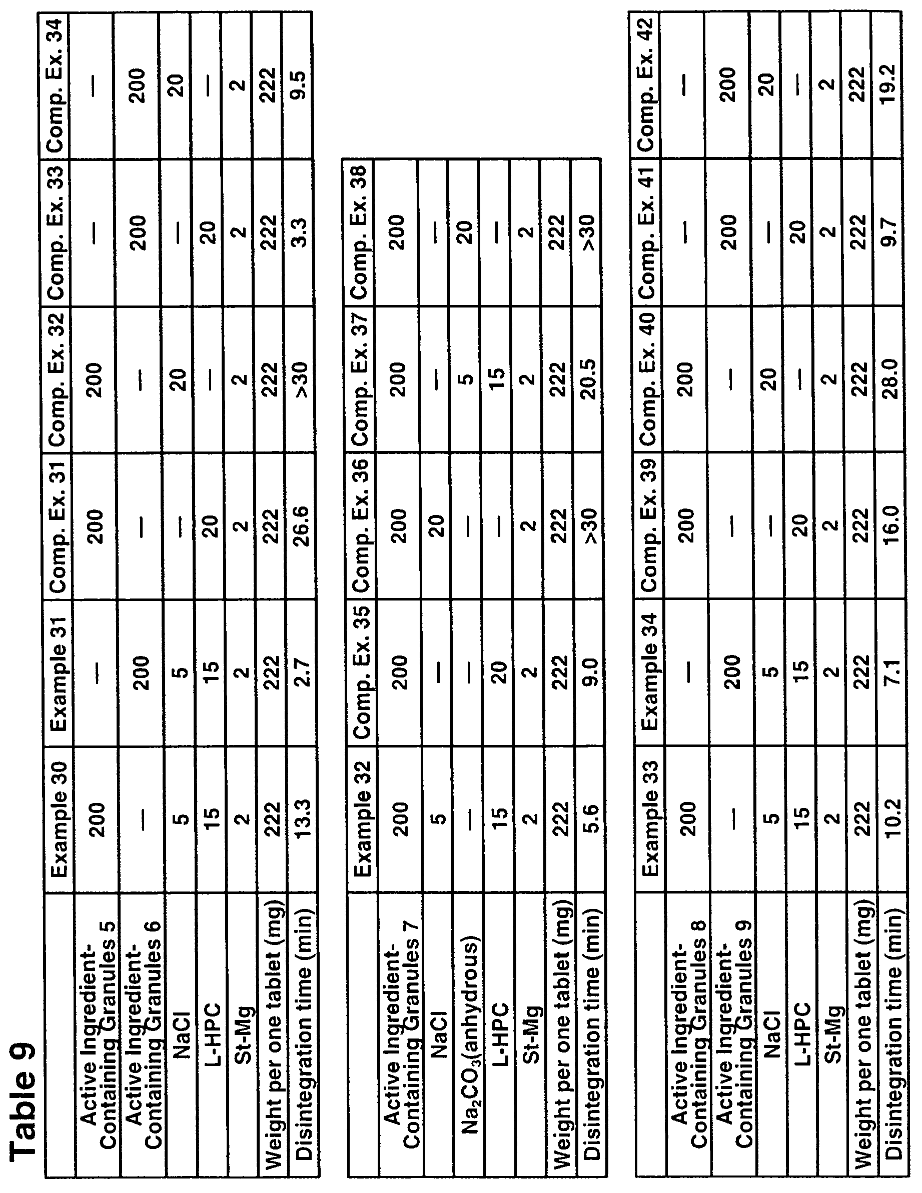

The additives mentioned in the Examples below are either substances that conform to official compendia, such as the Pharmacopoeia of Japan, Japanese Pharmaceutical Excipients 2003 and the Japanese Pharmaceutical Codex 1997, or reagents. In the following, Examples of the present invention and the Comparative Examples, in those case where the water-soluble salt was to be added in powder form to the formulation during or subsequent to the granulating step, the water-soluble salt was used after being finely ground in a mortar.

### Example 1

A suitable amount of purified water was added to 10 g of a dipeptidyl peptidase IV inhibitor (3-but-2-ynyl-5-methyl-2-piperazin-1-yl-3,5-dihydro-4H-imidazo[4,5-d]pyridazin-4-one tosylate; Eisai Inc.), 5 g of mannitol and 0.5 g of hydroxypropyl cellulose (HPC-L; Nippon Soda), and the ingredients were mixed in a mortar, then dried under heating in a thermostatic chamber, thereby giving Active Ingredient-Containing Granules 1. Next, 5 mg of sodium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 1 and mixed. An Autograph AG5000A (Shimadzu Corporation) was then used to compress the mixture into tablets under 1,200 kg of pressure, thereby giving tablets having an individual weight of 222 mg and a diameter of 8.5 mm.

### Example 2

10 mg of sodium chloride, 10 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith. An Autograph AG5000A (Shimadzu Corporation) was then used to compress the mixture into tablets under 1,200 kg of pressure, thereby giving tablets having an individual weight of 222 mg and a diameter of 8.5 mm.

### Example 3

A suitable amount of purified water was added to 77.80 g of a dipeptidyl peptidase IV inhibitor (3-but-2-ynyl-5-methyl-2-piperazin-1-yl-3,5-dihydro-4*H-*imidazo[4,5-d]pyridazin-4-one tosylate; Eisai Inc.), 8.92 g of mannitol, 14.10 g of cornstarch, 21.15 g of low-substituted hydroxypropyl cellulose (L-HPC LH21; Shin-Etsu Chemical) and 3.53 g of hydroxypropyl cellulose (HPC-L; Nippon Soda), and the mixture was granulated in a stirring granulator. The resulting granulated granules were dried under heating in a thermostatic chamber, then rendered to a uniform size, thereby giving Active Ingredient-Containing Granules 2. Next, 23.5 mg of microcrystalline cellulose, 1.2 mg of sodium chloride and 2.4 mg of magnesium stearate were added per 209.2 mg of the Active Ingredient-Containing Granules 2 and mixed therewith. An Autograph AG5000A (Shimadzu Corporation) was then used to compress the mixture into tablets under 1,200 kg of pressure, thereby giving tablets having an individual weight of 236.2 mg and a diameter of 8.5 mm.

### Example 4

Microcrystalline cellulose (23.5 mg), 2.4 mg of sodium chloride and 2.4 mg of magnesium stearate were added per 209.2 mg of the Active Ingredient-Containing Granules 2 prepared in Example 3 and mixed therewith. An Autograph AG5000A (Shimadzu Corporation) was then used to compress the mixture into tablets under 1,200 kg of pressure, thereby giving tablets having an individual weight of 237.4 mg and a diameter of 8.5 mm.

### Example 5

Microcrystalline cellulose (23.5 mg), 4.7 mg of sodium chloride and 2.4 mg of magnesium stearate were added per 209.2 mg of the Active Ingredient-Containing Granules 2 prepared in Example 3 and mixed therewith. An Autograph AG5000A (Shimadzu Corporation) was then used to compress the mixture into tablets under 1,200 kg of pressure, thereby giving tablets having an individual weight of 239.7 mg and a diameter of 8.5 mm.

### Example 6

Microcrystalline cellulose (23.5 mg), 11.8 mg of sodium chloride and 2.4 mg of magnesium stearate were added per 209.2 mg of the Active Ingredient-Containing Granules 2 prepared in Example 3 and mixed therewith. An Autograph AG5000A (Shimadzu Corporation) was then used to compress the mixture into tablets under 1,200 kg of pressure, thereby giving tablets having an individual weight of 246.8 mg and a diameter of 8.5 mm.

### Example 7

A suitable amount of purified water was added to 2.593 g of a dipeptidyl peptidase IV inhibitor (3-but-2-ynyl-5-methyl-2-piperazin-1-yl-3,5-dihydro-4*H-*imidazo[4,5-d]pyridazin-4-one tosylate; Eisai Inc.), 0.415 g of mannitol, 0.470 g of cornstarch, 0.705 g of low-substituted hydroxypropyl cellulose (L-HPC LH21; Shin-Etsu Chemical), 0.094 g of sodium chloride and 0.141 g of hydroxypropyl cellulose (HPC-L; Nippon Soda). The ingredients were mixed in a mortar, the mixture was then dried under heating in a thermostatic chamber, thereby giving Active Ingredient-Containing Granules 4. Next, 11.8 mg of microcrystalline cellulose and 2.4 mg of magnesium stearate were added per 220.9 mg of the Active Ingredient-Containing Granules 4 and mixed therewith. An Autograph AG-5000A (Shimadzu Corporation) was then used to compress the mixture into tablets under 1,200 kg of pressure, thereby giving tablets having an individual weight of 235.0 mg and a diameter of 8.5 mm.

Comparative Examples 1 to 7 are provided below so as to illustrate the remarkable effects of the pharmaceutical compositions obtained in the above Examples 1 to 7.

### Comparative Example 1

2 mg of magnesium stearate was added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 202 mg and a diameter of 8.5 mm.

### Comparative Example 2

10 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 212 mg and a diameter of 8.5 mm.

### Comparative Example 3

20 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 4

40 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 242 mg and a diameter of 8.5 mm.

### Comparative Example 5

20 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 6

40 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 242 mg and a diameter of 8.5 mm.

### Comparative Example 7

Microcrystalline cellulose (23.5 mg) and 2.4 mg of magnesium stearate were added per 209.2 mg of the Active Ingredient-Containing Granules 2 prepared in Example 3 and mixed therewith, an Autograph AG5000A (Shimadzu Corporation) was then used to compress the mixture into tablets under 1,200 kg of pressure, thereby giving tablets having an individual weight of 235.0 mg and a diameter of 8.5 mm.

### Comparative Example 8

Microcrystalline cellulose (23.5 mg), 11.8 mg of low-substituted hydroxypropyl cellulose (L-HPC LH21; Shin-Etsu Chemical) and 2.4 mg of magnesium stearate were added per 209.2 mg of the Active Ingredient-Containing Granules 2 prepared in Example 3 and mixed therewith, an Autograph AG5000A (Shimadzu Corporation) was then used to compress the mixture into tablets under 1,200 kg of pressure, thereby giving tablets having an individual weight of 246.8 mg and a diameter of 8.5 mm.

### Comparative Example 9

A suitable amount of purified water was added to 2.593 g of a dipeptidyl peptidase IV inhibitor (3-but-2-ynyl-5-methyl-2-piperazin-1-yl-3,5-dihydro-4*H-*imidazo[4,5-d]pyridazin-4-one tosylate; Eisai Inc.), 0.509 g of mannitol, 0.470 g of cornstarch, 0.705 g of low-substituted hydroxypropyl cellulose (L-HPC LH21; Shin-Etsu Chemical) and 0.141 g of hydroxypropyl cellulose (HPC-L; Nippon Soda). The ingredients were mixed in a mortar, the mixture was then dried under heating in a thermostatic chamber, thereby giving Active Ingredient-Containing Granules 3. Next, 11.8 mg of microcrystalline cellulose and 2.4 mg of magnesium stearate were added per 220.9 mg of the Active Ingredient-Containing Granules 3 and mixed therewith, an Autograph AG5000A (Shimadzu Corporation) was then used to compress the mixture into tablets under 1,200 kg of pressure, thereby giving tablets having an individual weight of 235.0 mg and a diameter of 8.5 mm.

### Example 8

10 mg of croscarmellose sodium (Ac-di-sol; FMC International), 10 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 9

15 mg of croscarmellose sodium (Ac-di-sol; FMC International), 5 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 10

10 mg of crospovidone (polyplasdone XL; ISP), 10 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 11

15 mg of crospovidone (polyplasdone XL; ISP), 5 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 12

10 mg of carboxymethylcellulose calcium (ECG-505; Nichirin Kagaku Kogyo), 10 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 13

15 mg of carboxymethylcellulose calcium (ECG-505; Nichirin Kagaku Kogyo), 5 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 14

10 mg of sodium carboxymethyl starch (EXPLOTAB; Kimura Sangyo), 10 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 15

15 mg of sodium carboxymethyl starch (EXPLOTAB; Kimura Sangyo), 5 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

Comparative Examples 10 to 15 are provided below so as to illustrate the remarkable effects of the pharmaceutical compositions obtained in the above Examples 8 to 15.

### Comparative Example 10

20 mg of croscarmellose sodium (Ac-di-sol; FMC International) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 11

20 mg of crospovidone (polyplasdone XL; ISP) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 12

20 mg of carboxymethylcellulose calcium (ECG-505; Nichirin Kagaku Kogyo) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 13

20 mg of sodium carboxymethyl starch (EXPLOTAB; Kimura Sangyo) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 14

15 mg of cornstarch, 5 mg of sodium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 15

20 mg of cornstarch and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 16

5 mg of magnesium chloride hexahydrate, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 17

10 mg of magnesium chloride hexahydrate, 10 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 18

5 mg of anhydrous calcium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 19

10 mg of anhydrous calcium chloride, 10 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 20

5 mg of anhydrous sodium bicarbonate, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 21

5 mg of anhydrous disodium hydrogen phosphate, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 22

5 mg of potassium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 23

5 mg of ammonium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 24

5 mg of anhydrous sodium acetate, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 25

5 mg of glycine, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

Comparative Examples 16 to 26 are provided below so as to illustrate the remarkable effects of the pharmaceutical compositions obtained in the above Examples 16 to 25.

### Comparative Example 16

5 mg of anhydrous sodium carbonate, 15 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 17

10 mg of anhydrous sodium carbonate, 10 mg of low-substituted hydroxypropyl cellulose (L-HPC LH21; Shin-Etsu Chemical) and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 18

20 mg of anhydrous sodium carbonate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 19

20 mg of magnesium chloride hexahydrate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 20

20 mg of anhydrous calcium chloride and 2 mg of magnesium stearate were added per 200 mg of the Active Ingredient-Containing Granules 1 prepared in Example 1 and mixed therewith, an Autograph AG-5000A (Shimadzu Corporation) was then used to compress the mixture into tablets under 1,200 kg of pressure, thereby giving tablets having an individual weight of 222 mg and a diameter of 8.5 mm.

### Comparative Example 21

20 mg of anhydrous sodium bicarbonate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 22

20 mg of anhydrous disodium hydrogen phosphate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 23

20 mg of potassium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 24

20 mg of ammonium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 25

20 mg of anhydrous sodium acetate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 26

20 mg of glycine and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 26

15 mg of croscarmellose sodium (Ac-di-sol; FMC International), 5 mg of ammonium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 27

15 mg of crospovidone (polyplasdone XL; ISP), 5 mg of ammonium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 28

15 mg of carboxymethylcellulose calcium (ECG-505; Nichirin Kagaku Kogyo), 5 mg of ammonium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 29

15 mg of sodium carboxymethyl starch (EXPLOTAB; Kimura Sangyo), 5 mg of ammonium chloride and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

Comparative Examples 27 to 30 are provided below so as to illustrate the remarkable effects of the pharmaceutical compositions obtained in the above Examples 26 to 29.

### Comparative Example 27

15 mg of croscarmellose sodium (Ac-di-sol; FMC International), 5 mg of anhydrous sodium carbonate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 28

15 mg of crospovidone (polyplasdone XL; ISP), 5 mg of anhydrous sodium carbonate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 29

15 mg of carboxymethylcellulose calcium (ECG-505; Nichirin Kagaku Kogyo), 5 mg of anhydrous sodium carbonate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 30

15 mg of sodium carboxymethyl starch (EXPLOTAB; Kimura Sangyo), 5 mg of anhydrous sodium carbonate and 2 mg of magnesium stearate were added per 200 mg of the Active Ingredient-Containing Granules 1 prepared in Example 1 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 30

A suitable amount of purified water was added to 2 g of the anti-anxiety drug E2508 prepared by the method described in International Publication WO 02/088121 (N-cyclopropylmethyl-7- (2,6-dimethoxy-4-methoxymethylphenyl) -2-ethyl-N-(tetrahydro-2H-pyran-4-ylmethyl)pyrazolo[1,5-a]pyridine-3-amine tosylate; Eisai Inc.), 2 g of mannitol and 0.12 g of hydroxypropyl cellulose (HPC-L; Nippon Soda), and the ingredients were mixed in a mortar, then dried under heating in a thermostatic chamber, thereby giving Active Ingredient-Containing Granules 5. Next, 5 mg of sodium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 5 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 31

A suitable amount of purified water was added to 3 g of ascorbic acid (Daiichi Pharmaceutical), which is a type of water-soluble vitamin, 1 g of mannitol and 0.12 g of hydroxypropyl cellulose (HPC-L; Nippon Soda), and the ingredients were mixed in a mortar, then dried under heating in a thermostatic chamber, thereby giving Active Ingredient-Containing Granules 6. Next, 5 mg of sodium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 6 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 32

A suitable amount of purified water was added to 2 g of glibenclamide (Wako Pure Chemical Industries), which is a sulfonylurea drug for treating diabetes, 2 g of mannitol and 0.12 g of hydroxypropyl cellulose (HPC-L; Nippon Soda), and the ingredients were mixed in a mortar, then dried under heating in a thermostatic chamber, thereby giving Active Ingredient-Containing Granules 7. Next, 5 mg of sodium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 7 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 33

A suitable amount of purified water was added to 1 g of donepezil hydrochloride (Eisai Inc.), which is an antidementia agent, 1 g of mannitol and 0.06 g of hydroxypropyl cellulose (HPC-L; Nippon Soda), and the ingredients were mixed in a mortar, then dried under heating in a thermostatic chamber, thereby giving Active Ingredient-Containing Granules 8. Next, 5 mg of sodium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 8 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Example 34

A suitable amount of purified water was added to 1 g of memantine hydrochloride (Lachema s.r.o.), which is an antidementia agent, 1 g of mannitol and 0.06 g of hydroxypropyl cellulose (HPC-L; Nippon Soda), and the ingredients were mixed in a mortar, then dried under heating in a thermostatic chamber, thereby giving Active Ingredient-Containing Granules 9. Next, 5 mg of sodium chloride, 15 mg of low-substituted hydroxypropyl cellulose (L-HPC LH21; Shin-Etsu Chemical) and 2 mg of magnesium stearate were added per 200 mg of the Active Ingredient-Containing Granules 9 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

Comparative Examples 31 to 42 are provided below so as to illustrate the remarkable effects of the pharmaceutical compositions obtained in the above Examples 30 to 34.

### Comparative Example 31

20 mg of low-substituted hydroxypropyl cellulose (L-HPC LH21; Shin-Etsu Chemical) and 2 mg of magnesium stearate were added per 200 mg of the Active Ingredient-Containing Granules 5 prepared in Example 30 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 32

20 mg of sodium chloride and 2 mg of magnesium stearate were added per 200 mg of the Active Ingredient-Containing Granules 5 prepared in Example 30 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 33

20 mg of low-substituted hydroxypropyl cellulose (L-HPC LH21; Shin-Etsu Chemical) and 2 mg of magnesium stearate were added per 200 mg of the Active Ingredient-Containing Granules 6 prepared in Example 31 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 34

20 mg of sodium chloride and 2 mg of magnesium stearate were added per 200 mg of the Active Ingredient-Containing Granules 6 prepared in Example 31 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 35

20 mg of low-substituted hydroxypropyl cellulose (L-HPC LH21; Shin-Etsu Chemical) and 2 mg of magnesium stearate were added per 200 mg of the Active Ingredient-Containing Granules 7 prepared in Example 32 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 36

20 mg of sodium chloride and 2 mg of magnesium stearate were added per 200 mg of the Active Ingredient-Containing Granules 7 prepared in Example 32 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 37

15 mg of low-substituted hydroxypropyl cellulose (L-HPC LH21; Shin-Etsu Chemical), 5 mg of anhydrous sodium carbonate and 2 mg of magnesium stearate were added per 200 mg of the Active Ingredient-Containing Granules 7 prepared in Example 32 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 38

20 mg of anhydrous sodium carbonate and 2 mg of magnesium stearate were added per 200 mg of the Active Ingredient-Containing Granules 7 prepared in Example 32 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 39

20 mg of low-substituted hydroxypropyl cellulose (L-HPC LH21; Shin-Etsu Chemical) and 2 mg of magnesium stearate were added per 200 mg of the Active Ingredient-Containing Granules 8 prepared in Example 33 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 40

20 mg of sodium chloride and 2 mg of magnesium stearate were added per 200 mg of the Active Ingredient-Containing Granules 8 prepared in Example 33 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 41

20 mg of low-substituted hydroxypropyl cellulose (L-HPC LH21; Shin-Etsu Chemical) and 2 mg of magnesium stearate were added per 200 mg of the Active Ingredient-Containing Granules 9 prepared in Example 34 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Comparative Example 42

20 mg of sodium chloride and 2 mg of magnesium stearate were added per 200 mg of the Active Ingredient-Containing Granules 9 prepared in Example 34 and mixed therewith, an Autograph AG5000A (Shimadzu Corporation) was then used to compress the mixture into tablets under 1,200 kg of pressure, thereby giving tablets having an individual weight of 222 mg and a diameter of 8.5 mm.

### Industrial Applicability

The present invention improves the disintegratability of the pharmaceutical compositions without increasing the size of the dosage form and without a decline in quality due to interactions between the pharmaceutically active ingredient and the disintegrator, and thus enables the production of the pharmaceutical compositions having a rapid disintegration time. Moreover, in the present invention, by using a premix composition which lacks a pharmaceutically active ingredient, and which comprises at least one disintegrant and at least one water-soluble salt having a pH being from 3 to 9 in an aqueous solution of 2.5% concentration, the pharmaceutical compositions of improved disintegratability can be easily produced by merely adding the premix composition to the formulation. Because improvements in disintegratability can thus be achieved without a loss in the quality of the drug product, the present invention has enormous potential in industry.

### Brief Description of Drawings

FIG. 1 shows the synergistic effects of the combined use of various disintegrants with sodium chloride on improving the disintegratability of tablets.
FIG. 2 shows the relationship between "the pH of 2.5 wt % aqueous solutions of various water-soluble salts" and "the disintegration time for tablets containing both those salts and L-HPC".
FIG. 3 shows the synergistic effects of the combined use of various disintegrants with ammonium chloride on improving the disintegratability of tablets.
FIG. 4 shows the absence of synergistic improvements in the disintegratability of tablets even with the combined use of various disintegrants with anhydrous sodium carbonate.

## Claims

1. A method for preparing a pharmaceutical composition, comprising:
blending, in the pharmaceutical composition containing a pharmaceutically active ingredient, at least one disintegrant and at least one water-soluble salt having a pH being from 3 to 9 in an aqueous solution of 2.5% concentration.

2. The method according to Claim 1, wherein the water-soluble salt is a water-soluble inorganic salt.

3. The method according to Claim 2, wherein the water-soluble inorganic salt is selected from the group consisting of sodium chloride, magnesium chloride, sodium bicarbonate, potassium chloride and ammonium chloride.

4. The method according to Claim 2, wherein the water-soluble inorganic salt is sodium chloride.

5. The method according to any one of Claims 1 to 4, wherein the disintegrant is selected from the group consisting of croscarmellose sodium, crospovidone, carboxymethylcellulose calcium, low-substituted hydroxypropyl cellulose, and sodium carboxymethyl starch.

6. The method according to any one of Claims 1 to 4, wherein the disintegrant is low-substituted hydroxypropyl cellulose.

7. The method according to any one of Claims 1 to 6, wherein the disintegrant is blended in the pharmaceutical composition in an amount of from 2 to 15 % by weight.

8. The method according to any one of Claims 1 to 7, wherein the water-soluble salt is blended in an amount of from 0.05 to 2 parts by weight based on one part by weight of the disintegrant.

9. The method according to any one of Claims 1 to 8, wherein the pharmaceutically active ingredient is blended in the pharmaceutical composition in an amount of from 20 to 97 % by weight.

10. A method for preparing a pharmaceutical composition, comprising:
blending, in the pharmaceutical composition containing a pharmaceutically active ingredient, at least low-substituted hydroxypropyl cellulose and sodium chloride.

11. A premix composition, comprising:
at least one disintegrant; and
at least one water-soluble salt having a pH being from 3 to 9 in an aqueous solution of 2.5% concentration;
wherein the premix composition lacks a pharmaceutically active ingredient.

12. The premix composition according to Claim 11, wherein the water-soluble salt is a water-soluble inorganic salt.

13. The premix composition according to Claim 12, wherein the water-soluble inorganic salt is selected from the group consisting of sodium chloride, magnesium chloride, sodium bicarbonate, potassium chloride and ammonium chloride.

14. The premix composition according to Claim 12, wherein the water-soluble inorganic salt is sodium chloride.

15. The premix composition according to any one of Claims 11 to 14, wherein the disintegrant is selected from the group consisting of croscarmellose sodium, crospovidone, carboxymethylcellulose calcium, low-substituted hydroxypropyl cellulose, and sodium carboxymethyl starch.

16. The premix composition according to any one of Claims 11 to 14, wherein the disintegrant is low-substituted hydroxypropyl cellulose.

17. The premix composition according to any one of Claims 11 to 16, wherein the water-soluble salt is included in an amount of from 0.05 and 2 parts by weight based on one part by weight of the disintegrant.

18. A premix composition, comprising:
at least low-substituted hydroxypropyl cellulose; and
sodium chloride,
wherein the premix composition lacks a pharmaceutically active ingredient.

19. A method for improving disintegratability of a pharmaceutical composition, comprising:
blending, in the pharmaceutical composition containing a pharmaceutically active ingredient, at least one disintegrant and at least one water-soluble salt having a pH being from 3 to 9 in an aqueous solution of 2.5% concentration.
